# EUROPEAN PATENT SPECIFICATION

(11) **EP 3 003 468 B1**
(45) Date of publication and mention of the grant of the patent: **28.08.2019**
(21) Application number: 14733824.8
(22) Date of filing: 23.05.2014
(51) Int. Cl.: A61N 1/05

(54) **METHODS FOR MANUFACTURING SEGMENTED ELECTRODE LEADS USING A REMOVABLE RING AND THE LEADS FORMED THEREBY**
VERFAHREN ZUR HERSTELLUNG SEGMENTIERTER ELEKTRODENLEITUNGEN MIT EINEM ABNEHMBAREN RING UND IN DIESEM VERFAHREN HERGESTELLTE LEITUNGEN
PROCÉDÉ DE FABRICATION DE CONDUCTEURS D'ÉLECTRODES SEGMENTÉES AU MOYEN D'UN ANNEAU AMOVIBLE ET CONDUCTEURS FORMÉS PAR CELUI-CI

(30) Priority: 31.05.2013 US 201361829912 P
(43) Date of publication of application: 13.04.2016
(73) Proprietor: Boston Scientific Neuromodulation Corporation, Valencia, CA 91355 (US)
(72) Inventor: HOWARD, Joshua, Dale, Winnetka, CA 91306 (US); PIANCA, Anne, Margaret, Santa Monica, CA 90403 (US)
(74) Representative: Vossius & Partner Patentanwälte Rechtsanwälte mbB
(86) International application number: PCT/US2014/039453
(87) International publication number: WO 2014/193761

(56) References cited:
- US-A1- 2010 269 339
- US-A1- 2011 245 903
- US-A1- 2011 313 500

## Description

### CROSS-REFERENCE TO RELATED APPLICATIONS

This application claims the benefit under 35 U.S.C. § 119(c) of U.S. Provisional Patent Application Serial No. 61/829,912, filed May 31, 2013.

### FIELD

The present invention is directed to the area of implantable electrical stimulation systems and methods of making and using the systems. The present invention is also directed to implantable electrical stimulation leads having segmented electrodes, as well as methods of making and using the tends and electrical stimulation systems.

### BACKGROUND

Electrical stimulation can be useful for treating a variety of conditions. Deep brain stimulation can be useful for treating, for example, Parkinson's disease, dystonia, essential tremor, chronic pain, Huntington's disease, levodopa-induced dyskinesias and rigidity, bradykinesia, epilepsy and seizores, eating disorders, and mood disorder. Typically, a lead with a stimulating electrode at or near a tip of the lead provides the stimulation to target neurons in the brain. Magnetic resonance imaging ("MRI") or computerized tomography ("CT") scans can provide a starting point for determining where the stimulating electrode should be positioned to provide the desired stimulus to the target neurons.

After the lead is implanted into a patient's brain, electrical stimulus current can be delivered through selected electrodes on the lead to stimulate target neurons in the brain. Typically, the electrodes are formed into rings disposed on a distal portion of the lead.

The stimulus current projects from the ring electrodes equally in every direction. Because of the ring shape of these electrodes, the stimulus current cannot be directed to one or more specific positions around the ring electrode (*e.g.*, on one or more sides, or points, mound the lead). Consequently, undirected stimulation may result in unwanted stimulation of neighboring neural tissue, potentially resulting in undesired side effects US 2011/0245903 A1 relates to leads having directional electrodes as well as retention ledges to secure the electrodes to the leads. Methods of manufacturing leads and methods of treating conditions and selectively stimulating regions of the nervous system are also provided.

US 2010/0269339 A1 relates to a process for fabricating a medical lead comprising: providing a splicing tube having a plurality of angularly spaced longitudinal grooves; placing a first plurality of conductors within the plurality of angularly spaced grooves defined on an exterior surface of the splicing tube; placing a second plurality of conductors within the plurality of angularly spaced grooves and adjacent to the first plurality of conductors such that a portion of the distal ends overlap a portion of the proximal ends; positioning conductive filler material adjacent to the overlapped portions of the distal and proximal ends; electrically coupling the proximal ends of the first plurality of electrodes to respective proximal ends of the second plurality of electrodes; molding insulative material about at least the electrode assembly and the splicing tube; and fusing the splicing tube with the insulative material from the molding and the lead body.

### BRIEF SUMMARY

One embodiment is a method of making an electrical stimulation lead. The method includes attaching segmented electrodes to an interior of a ring in a circumferentially spaced-apart arrangement; attaching a conductor wire to each of the segmented electrodes; coupling the ring with the segmented electrodes to a lead body; and, alter coupling to the lead body, removing at least those portions of the ring between the segmented electrodes to separate the plurality of segmented electrodes from each other.

Another embodiment is a pre-electrode that includes a ring having an interior; and segmented electrodes attached to the interior of the ring in a circumferentially spaced-apart arrangement.

Yet another embodiment is a method of making a pre-electrode. The method includes placing a portion of tool in a ring where the portion of the tool defines channels for receiving segmented electrodes; individually inserting segmented electrodes into the channels of the tool and sliding the segmented electrodes into the ring; attaching the segmented electrodes to an interior of the ring in a circumferentially spaced-apart arrangement defined by the tool: and removing the

The invention is defined in claims 1 and 10. Further aspects and preferred embodiments are defined in the dependent claims. Aspects, embodiments and examples of the present disclosure which do not fall under the scope of the appended claims do not form part of the invention and are merely provided for illustrative purposes.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic side view of one embodiment of a device for brain stimulation, according to the invention;
FIG. 2 is a schematic diagram of radial current steering along various electrode levels along the length of a lead, according to the invention;
FIG. 3A is a perspective view of an embodiment of a portion of a lead having a plurality of segmented electrodes, according to the invention;
FIG. 3B is a perspective view of a second embodiment of a portion of a lead having a plurality of segmented electrodes, according to the invention;
FIG. 3C is a perspective view of a third embodiment of a portion of a lead having a plurality of segmented electrodes, according to the invention;
FIG. 3D is a perspective view of a fourth embodiment of a portion of a lead having a plurality of segmented electrodes, according to the invention;
FIG. 3E is a perspective view of a firth embodiment of a portion of a lead having a plurality of segmented electrodes, according to the invention;
FIG. 3F is a perspective view of a sixth embodiment of a portion of a lead having a plurality of segmented electrodes, according to the invention;
FIG. 3G is a perspective view of a seventh embodiment of a portion of a lead having a plurality of segmented electrodes, according to the invention;
FIG. 4 is a perspective view of one embodiment of a ring with segmented electrodes attached to an interior thereof;
FIG. 5 is a perspective view of one embodiment of a segmented electrode, according to the invention;
FIG. 6A is a side view of one embodiment of tool for placing the segmented electrodes within a ring, according to the invention;
FIG. 6B is a cross-sectional view of the tool of FIG. 6A along line 6B-6B. according to the invention;
FIG. 6C is a cross-sectional view of one embodiment of a ring with segmented electrodes and the tool of FIG. 6A disposed therein;
FIG. 7A is a cross-sectional view of one embodiment of a pre-etectrode including a ring and segmented electrodes attached to an interior thereof;
FIG. 7B is a cross-sectional view of the pre-electrode of FIG. 7A with a portion of a lead body formed therein;
FIG. 7C is a cross-sectional view of one embodiment of a lead with segmented electrodes formed from the pre-electrode of FIGS. 7A and 7B, according to the mention;
FIG. 8A is a cross-sectional view of one embodiment of a pre-electrode including a ring having an opening and segmented electrodes attached to an interior thereof, according to the invention;
FIG. 8B is a side view of the ring of FIG. 8A where the opening is a slit; and
FIG. 8C is a side view of the ring of FIG. 8A where the opening is a set of holes through the ring, according to the invention.

### DETAILED DESCRIPTION

The present invention is directed to the area of implantable electrical stimulation systems and methods of making and using the systems. The present invention is also directed to implantable electrical stimulation leads having segmented electrodes, as well as methods of making and using the leads and electrical stimulation systems.

A lead for deep brain stimulation may include stimulation electrodes, recording electrodes, or a combination of both. At least some of the stimulation electrodes, recording electrodes, or both are provided in the form of segmented electrodes that extend only partially around the circumference of the lead. These segmented electrodes can be provided in sets of electrodes, with each set having electrodes radially distributed about the lead at a particular longitudinal position. For illustrative purposes, the leads are described herein relative to use for deep brain stimulation, but it will be understood that any of the leads can be used for applications other than deep brain stimulation, including spinal cord stimulation, peripheral nerve stimulation, or stimulation of other nerves and tissues.

Suitable implantable electrical stimulation systems include, but are not limited to a least one lead with one or more electrodes disposed on a distal end of the lead and one or more terminals disposed on one or more proximal ends of the lead. Leads include, for example, percutaneous leads. Examples of electrical stimulation systems with leads are found in, for example, U.S. Patents Nos. 6,181,909; 6,516,227; 6,609,029, 0,609,032; 6,741,892; 7,244,150; 7,450,997; 7,672,734;7,761,165; 7,783,359; 7,792,590; 7,809,446; 7,949,395; 7,974,706; 8,175,710; 8,224,450; 8,271,094; 8,295,944; 8,364,278; 8,391,985; and 8,688,235; and U.S. Patent Applications Publication Nos. 2007/0150036; 2009/0187222; 2009/0276021; 2010/0076535; 2010/0268298; 2011/0005069; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203311; 2012/0316615; 2013/0105071; and 2013/0197602.

In at least some embodiments, a practitioner may determine the position of the target neurons using recording electrode(s) and then position the stimulation electrode(s) accordingly. In some embodiments, the same electrodes can be used for both recording and stimulation. In some embodiments, separate leads can be used; one with recording electrodes which identity target neurons, and a second lead with stimulation electrodes that replaces the first after target neuron identification. In some embodiments, the same lead may include both recording electrodes and stimulation electrodes or electrodes may be used for both recording and stimulation.

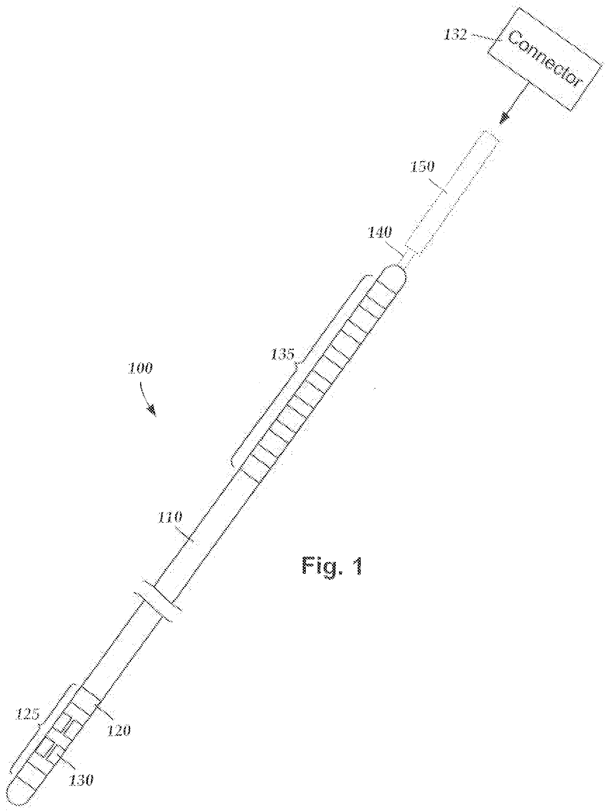

Figure 1 illustrates one embodiment of a device 100 for brain stimulation. The device includes a lead 110, a plurality of electrodes 125 disposed at least partially about a circumference of the lead 110, a plurality of terminals 135, a connector 132 for connection of the electrodes to a control unit, and a stylet 140 for assisting in insertion and positioning of the lead in the patient's brain. The stylet 140 can be made of a rigid material. Examples of suitable materials for the stylet include, but are not limited to, tungsten, stainless steel, and plastic. The stylet 140 may have a handle 150 to assist insertion into the lead 110, as well as rotation of the stylet 140 and lead 110. The connector 132 fits over a proximal end of the lead 110, preferably after removal of the stylet 140.

The control unit (not shown) is typically an implantable pulse generator that can be implanted into a patient's body, for example, below the patient's clavicle area. The pulse generator can have eight stimulation channels which may be independently programmable to control the magnitude of the current stimulus from each channel. In some cases the pulse generator may have more or fewer than eight stimulation channels (e.g., 4-, 6-, 16-, 32-, or more stimulation channels). The control unit may have one, two, three, four, or more connector ports, for receiving the plurality of terminals 135 at the proximal end of the lead 110.

In one example of operation, access to the desired position in the brain can be accomplished by drilling a hole in the patient's skull or cranium with a cranial drill (commonly referred to as a burr), and coagulating and incising the dura mater, or brain covering. The lead 110 can be inserted into the cranium and brain tissue with the assistance of the stylet 140. The lead 110 can be guided to the target location within the brain using, for example, a stereotactic frame and a microdrive motor system. In some embodiments, the microdrive motor system can be fully or partially automatic. The microdrive motor system may be configured to perform one or more the following actions (alone or in combination): insert the lead 110, retract the lead 110, or rotate the lead 110.

In some embodiments, measurement devices coupled to the muscles or other tissues stimulated by the target neurons, or a unit responsive to the patient or clinician, can be coupled to the control unit or microdrive motor system. The measurement device, user, or clinician can indicate a response by the target muscles or other tissues to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in tremor frequency or amplitude in response to stimulation of neurons. Alternatively, the patient or clinician may observe the muscle and provide feedback.

The lead 110 for deep brain stimulation can include stimulation electrodes recording electrodes, or both. In at least some embodiments, the lead 110 is rotatable so that the stimulation electrodes can be aligned with the target neurons after the neurons have been located using the recording electrodes.

Stimulation electrodes may be disposed on the circumference of the lead 110 to stimulate the target neurons. Stimulation electrodes may be ring-shaped so that current projects from each electrode equally in every direction from the position of the electrode along a length of the lead 110. Ring electrodes typically do not enable stimulus current to be directed from only a limited angular range around of the lead. Segmented electrodes, however, can be used to direct stimulus current to a selected angular range around the lead. When segmented electrodes are used in conjunction with an implantable pulse generator that delivers constant current stimulus, current steering can be achieved to more precisely deliver the stimulus to a position around an axis of the lead (*i.e.,* radial positioning around the axis of the lead).

To achieve current steering, segmented electrodes can be utilized in addition to, or as an alternative to, ring electrodes. Though the following description discusses stimulation electrodes, it will be understood that all configurations of the stimulation electrodes discussed may be utilized in arranging recording electrodes as well.

The lead 100 includes a lead body 110, one or more optional ring electrodes 120, and a plurality of sets of segmented electrodes 130. The lead body 110 can be formed of a biocompatible, non-conducting material such as, for example, a polymeric material. Suitable polymeric materials include, but are not limited to, silicone, polyurethane, polyurea, polyurethane-urea, polyethylene, or the like. Once implanted in the body, the lead 100 may be in contact with body tissue for extended periods of time. In at least some embodiments, the lead 100 has a cross-sectional diameter of no more than 1.5 mm and may be in the range of 0.5 to 1.5 mm. In at least some embodiments, the lead 100 has a length of at least 10 cm and the length of the lead 100 may be in the range of 10 to 70 cm.

The electrodes may be made using a metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes are made of a material that is biocompatible and does not substantially corrode under expected operating conditions in the operating environment for the expected duration of use.

Each of the electrodes can either be used or unused (OFF). When the electrode is used, the electrode can be used as an anode or cathode and carry anodic or cathodic current. In some instances, an electrode might be an anode for a period of time and a cathode for a period of time.

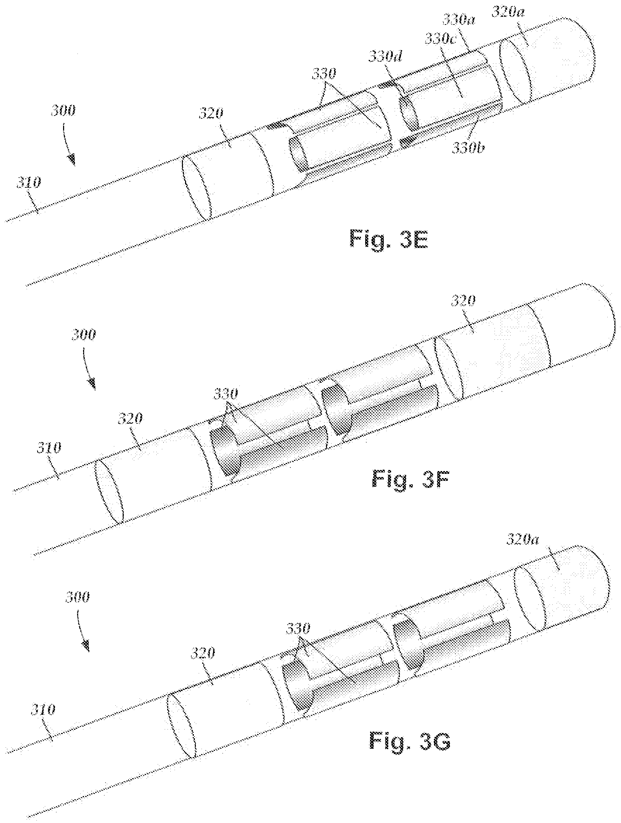

Stimulation electrodes in the form of ring electrodes 120 may be disposed on any part of the lead body 110, usually near a distal end of the lead 100. In Figure 1, the lead 100 includes two ring electrodes 120. Any number of ring electrodes 120 may be disposed along the length of the lead body 110 including, for example, one, two three, four, five, six, seven, eight, nine, ten, eleven, twelve, thirteen, fourteen, fifteen, sixteen or more ring electrodes 120. It will be understood that any number of ring electrodes may be disposed along the length of the lead body 110. In some embodiments, the ring electrodes 120 are substantially cylindrical and wrap around the entire circumference of the lead body 110. In some embodiments, the outer diameters of the ring electrodes 120 are substantially equal to the outer diameter of the lead body 110. The length of the ring electrodes 120 may vary according to the desired treatment and the location of the target neurons. In some embodiments the length of the ring electrodes 120 are less than or equal to the diameters of the ring electrodes 120. In other embodiments, the lengths of the ring electrodes 120 are greater than the diameters of the ring electrodes 120. The distal-most ring electrode 120 may be a tip electrode (see, e.g., tip electrode 320a of Figure 3E) which covers most, or all, of the distal tip of the lead.

Deep brain stimulation leads may include one or more sets of segmented electrodes. Segmented electrodes may provide for superior current steering than ring electrodes because target structures in deep brain stimulation are not typically symmetric about the axis of the distal electrode array. Instead, a target may be located on one side of a plane running through the axis of the lead. Through the use of a radially segmented electrode array ("RSEA"), current steering can be performed not only along a length of the lead but also around a circumference of the lead. This provides precise three-dimensional targeting and delivery of the current stimulus to neural target tissue, while potentially avoiding stimulation of other tissue. Examples of leads with segmented electrodes include U.S. Patent Application Publication Nos. 2010/0268298; 2011/0005069; 2011/0130803; 2011/0130816; 2011/0130817; 2011/0130818; 2011/0078900; 2011/0238129; 2012/0016378; 2012/0046710; 2012/0071949; 2012/0165911; 2012/197375; 2012/0203316; 2012/0203320; 2012/0203321.

In Figure 1, the lead 100 is shown having a plurality of segmented electrodes 130. Any number of segmented electrodes 130 may be disposed on the lead body 110 including, for example, one, two three, four, five, six, seven, eight, nine, ten, eleven, twelve, thirteen, fourteen, fifteen, sixteen or more segmented electrodes 130. It will be understood that any number of segmented electrodes 130 may be disposed along the length of the lead body 110. A segmented electrode 130 typically extends only 75%, 67%, 60%, 50%, 40%, 33%, 25%, 20%, 17%, 15%, or less around the circumference of the lead.

The segmented electrodes 130 may be grouped into sets of segmented electrodes, where each set is disposed around a circumference of the lead 100 at a particular longitudinal portion of the lead 100. The lead 100 may have any number segmented electrodes 130 in a given set of segmented electrodes. The lead 100 may have one, two, three, four, five, six, seven, eight, or more segmented electrodes 130 in a given set. In at least some embodiments, each set of segmented electrodes 130 of the lead 100 contains the same number of segmented electrodes 130. The segmented electrodes 130 disposed on the lead 100 may include a different number of electrodes than at least one other set of segmented electrodes 130 disposed on the lead 100.

The segmented electrodes 130 may vary in size and shape. In some embodiments, the segmented electrodes 130 are all of the same size, shape, diameter, width or area or any combination thereof. In some embodiments, the segmented electrodes 130 of each circumferential set (or even all segmented electrodes disposed on the lead 100) may be identical in size and shape.

Each set of segmented electrodes 130 may be disposed around the circumference of the lead body 110 to form a substantially cylindrical shape around the lead body 110. The spacing between individual electrodes of a given set of the segmented electrodes may be the same, or different from, the spacing between individual electrodes of another set of segmented electrodes on the lead 100. In at least some embodiments, equal spaces, gaps or cutouts are disposed between each segmented electrode 130 around the circumference of the lead body 110. In other embodiments, the spaces, gaps or cutouts between the segmented electrodes 130 may differ in size or shape. In other embodiments, the spaces, gaps, or cutouts between segmented electrodes 130 may be uniform tor a particular set of the segmented electrodes 130, or for all sets of the segmented electrodes 130. The sets of segmented electrodes 130 may be positioned in irregular or regular intervals along a length the lead body 110.

Conductor wires that attach to the ring electrodes 120 or segmented electrodes 130 extend along the lead body 110. These conductor wires may extend through the material of the lead 100 or along one or more lumens defined by the lead 100, or both. The conductor wires are presented at a connector (via terminals) for coupling of the electrodes 120, 130 to a control unit (not shown).

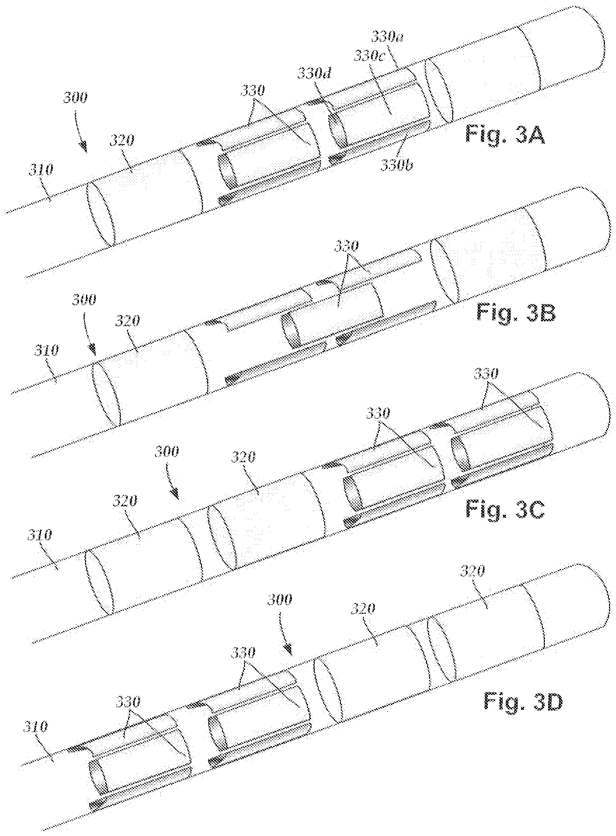

When the lead 100 includes both ring electrodes 120 and segmented electrodes 130, the ring electrodes 120 and the segmented electrodes 130 may be arranged in any suitable configuration. For example, when the lead 100 includes two sets of ring electrodes 120 and two sets of segmented electrodes 130, the ring electrodes 120 can flank the two sets of segmented electrodes 130 (see *e.g*., Figure 1). Alternately, the two sets of ring electrodes 120 can be disposed proximal to the two sets of segmented electrodes 130 (see *e.g*., Figure 3C), or the two sets of ring electrodes 120 can be disposed distal to the two sets of segmented electrodes 130 (see *e.g*., Figure 3D). One of the ring electrodes can be a tip electrode (see, tip electrode 320a of Figures 3E and 3G). It will be understood that other configurations are possible as well (*e.g*., alternating ring and segmented electrodes, or the like).

By varying the location of the segmented electrodes 130, different coverage of the target neurons may be selected. For example, the electrode arrangement of Figure 3C may be useful if the physician anticipates that the neural target will be closer to a distal tip of the lead body 110, while the electrode arrangement of Figure 3D may be useful if the physician anticipates that the neural target will be closer to a proximal end of the lead body 110.

Any combination of ring electrodes 120 and segmented electrodes 130 may be disposed on the lead 100. For example, the lead may include a first ring electrode 120. two sets of segmented electrodes; each set formed of four segmented electrodes 130, and a final ring electrode 120 at the end of the lead. This configuration may simply be referred to as a 1-4-4-1 configuration (Figures 3A and 3E). It may be useful to refer to the electrodes with this shorthand notation. Thus, the embodiment of Figure 3C may be referred to as a 1-1-4-4 configuration, while the embodiment of Figure 3D may be referred to as a 4-4-1-1 configuration. The embodiments of Figures 3F and 3G can be referred to as a 1-3-3-1 configuration. Other electrode configurations include, for example, a 2-2-2-2 configuration, where four sets of segmented electrodes are disposed on the lead, and a 4-4 configuration, where two sets of segmented electrodes, each having four segmented electrodes 130 are disposed on the lead. The 1-3-3-1 electrode configuration of Figures 3F and 3G has two sets of segmented electrodes, each set containing three electrodes disposed around the circumference of the lead, flanked by two ring electrodes (Figure 3F) or a ring electrode and a tip electrode (Figure 3G). In some embodiments, the lead includes 16 electrodes. Possible configurations for a 16-electrode lead include, but are not limited to 4-4-4-4; 8-8; 3-3-3-3-3-1 (and all rearrangements of this configuration); and 2-2-2-2-2-2-2-2.

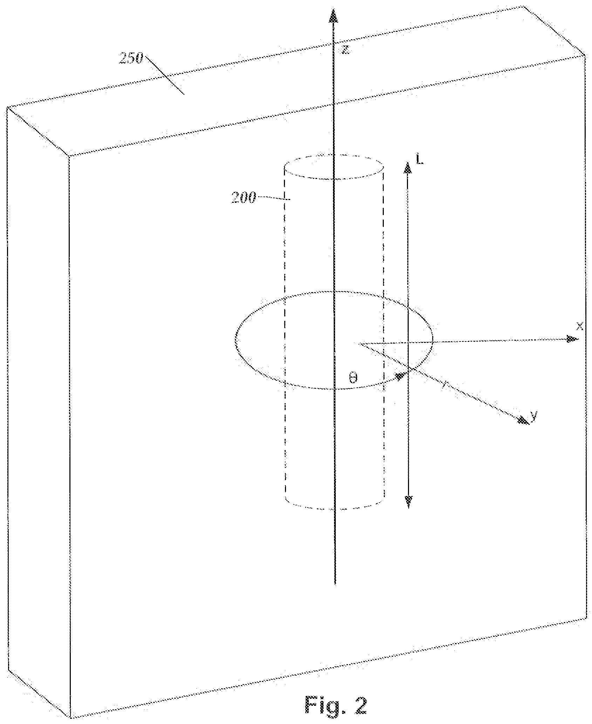

Figure 2 is a schematic diagram to illustrate radial current steering along various electrode levels along the length of the lead 200. While conventional lead configurations with ring electrodes are only able to steer current along the length of the lead (the *z*-axis), the segmented electrode configuration is capable of steering current in the *x*-axis, *y*-axis as well as the *z*-axis. Thus, the centroid of stimulation may be steered in any direction in the three-dimensional space surrounding the lead 200. In some embodiments, the radial distance, *r*, and the angle 0 around the circumference of the lead 200 may be dictated by the percentage of anodic current (recognizing that stimulation predominantly occurs near the cathode, although strong anodes may cause stimulation as well) introduced to each electrode. In at least some embodiments, the configuration of anodes and cathodes along the segmented electrodes allows the centroid of stimulation to be shifted to a variety of different locations along the lead 200.

As can be appreciated from Figure 2, the centroid of stimulation can be shifted at each level along the length of the lead 200. The use of multiple sets of segmented electrodes at different levels along the length of the lead allows for three-dimensional current steering. In some embodiments, the sets of segmented electrodes are shifted collectively (*i.e*., the centroid of simulation is similar at each level along the length of the lead). In at least some other embodiments, each set of segmented electrodes is controlled independently. Each set of segmented electrodes may contain two, three, four, five, six, seven, eight or more segmented electrodes. It will be understood that different stimulation profiles may be produced by varying the number of segmented electrodes at each level. For example, when each set of segmented electrodes includes only two segmented electrodes, uniformly distributed gaps (inability to stimulate selectively) may be formed in the stimulation profile. In some embodiments, at least three segmented electrodes 230 in a set are utilized to allow for true 360° selectivity.

As previously indicated, the foregoing configurations may also be used while utilizing recording electrodes, In some embodiments, measurement devices coupled to the muscles or other tissues stimulated by the target neurons or a unit responsive to the patient or clinician can be coupled to the control unit or microdrive motor system. The measurement device, user, or clinician can indicate a response by the target muscles or other tissues to the stimulation or recording electrodes to further identify the target neurons and facilitate positioning of the stimulation electrodes. For example, if the target neurons are directed to a muscle experiencing tremors, a measurement device can be used to observe the muscle and indicate changes in tremor frequency or amplitude in response to stimulation of neurons. Alternatively, the patient or clinician may observe the muscle and provide feedback.

The reliability and durability of the lead will depend heavily on the design and method of manufacture. Fabrication techniques discussed below provide methods that can produce manufacturable and reliable leads.

Returning to Figure 1, when the lead 100 includes a plurality of sets of segmented electrodes 130, it may be desirable to form the lead 100 such that corresponding electrodes of different sets of segmented electrodes 130 are radially aligned with one another along the length of the lead 100 (see *e.g*., the segmented electrodes 130 shown in Figure 1). Radial alignment between corresponding electrodes of different sets of segmented electrodes 130 along the length of the lead 100 may reduce uncertainty as to the location or orientation between corresponding segmented electrodes of different sets of segmented electrodes. Accordingly, it may be beneficial to form electrode arrays such that corresponding electrodes of different sets of segmented electrodes along the length of the lead 100 are radially aligned with one another and do not radially shift in relation to one another during manufacturing of the lead 100.

In other embodiments, individual electrodes in the two sets of segmented electrodes 130 are staggered (see, Figure 3B) relative to one another along the length of the lead body 110. In some cases, the staggered positioning of corresponding electrodes of different sets of segmented electrodes along the length of the lead 100 may be designed for a specific application.

Segmented electrodes can be used to tailor the stimulation region so that, instead of stimulating tissue around the circumference of the lead as would be achieved using a ring electrode, the stimulation region can be directionally targeted. In some instances, it is desirable to target a parallelepiped (or slab) region 250 that contains the electrodes of the lead 200, as illustrated in Figure 2. One arrangement for directing a stimulation field into a parallelepiped region uses segmented electrodes disposed on opposite sides of a lead.

Figures 3A-3E illustrate leads 300 with segmented electrodes 330, optional ring electrodes 320 or tip electrodes 320a, and a lead body 3 10. The sets of segmented electrodes 330 include either two (Figure 3B) or four (Figures 3A, 3C, and 3D) or any other number of segmented electrodes including, for example, three, five, six, or more.

Any other suitable arrangements of segmented electrodes can be used. As an example, arrangements in which segmented electrodes are arranged helically with respect to each other. One embodiment includes a double helix.

One challenge to making leads with segmented electrodes is the correct placement of the electrodes, and retention of the desired electrode placement, during the manufacturing process. In at least some embodiments, each set of segmented electrodes can be arranged by coupling the segmented electrodes of the set into a ring in a desired circumferential arrangement to form a pre-electrode. The pre-electrode can be disposed on the lead and a lead body formed around the segmented electrodes. After forming the lead body, the ring, or at least the portions of the ring between the segmented electrodes, can be removed to separate the segmented electrodes.

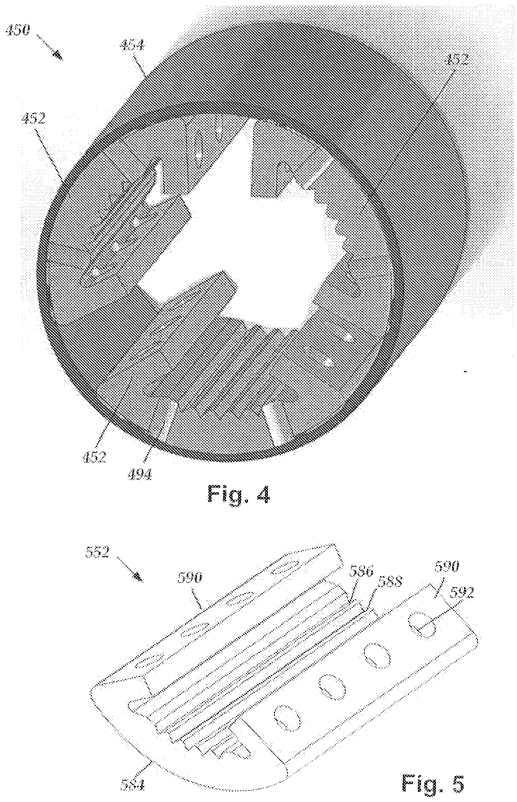

Figure 4 illustrates one embodiment of a pre-electrode 450 with three segmented electrodes 452 attached to an interior of a ring 454. Although the Figure illustrates three segmented electrodes, it will be understood that any other number of segmented electrodes may be provided within the ring including, but not limited to, two, three, four, five, six, or more segmented electrodes. In at least some embodiments, the segmented electrodes 452 are evenly or uniformly spaced-apart around the circumference of the ring 454, although other arrangements of the electrodes, including those in which the spacing is not uniform or even, are also acceptable.

The ring can have any suitable thickness. In at least some embodiments, the ring has a thickness no greater than 0.25 mm.

The electrodes 452 can be attached to the ring 454 in any suitable manner including, but not limited to, welding, soldering, using an adhesive, or any combination thereof. The ring 454 can be made of any suitable material including, but not limited to, metal, ceramic, or plastic materials, or any combination thereof. The ring 454 may be conductive or non-conductive. In at least some embodiments, the ring is made of a biocompatible material as part of the ring may be in the final lead or because processing of the ring may result in microseopic particles of the ring remaining in the lead even though the entire ring is intended to be removed.

The segmented electrodes can be formed in any suitable shape or size and can be formed of the materials described above. Figure 5 illustrates one example of a segmented electrode 552. In at least some embodiments, the segmented electrodes have a curved shape. The curved shape preferably corresponds to the curvature of the lead. For example, the curved shape of the segmented electrodes can have an arc of at least 10, 15, 20, 30, 40, 50, or 60 degrees. The arc of the segmented electrode may be no more than 175, 160, 150, 125, 115, 100, or 90 degrees. In some instance, the are of the segmented electrodes is in the range οf 10 to 175 degrees or in the range of 30 to 120 degrees or in the range of 40 to 100 degrees. The illustrated embodiments include three electrodes 452 disposed in the ring 454, but it will be recognized that any number of electrodes could be disposed within the ring including two, four, five, six, or more electrodes. Examples of other segmented electrodes that could be attached to the ring are presented in U.S. Provisional Patent Applications Serial Nos 61/829,908, and 61/829,918, both tiled May 31, 2013.

The segmented electrodes 552 optionally include one or more additional features to aid in holding the segmented electrode within the lead. One embodiment of a segmented electrode 552 displaying several optional features is provided in Figure 5. The segmented electrode includes a stimulation surface 584 that, when the lead is formed and inserted into the patient, will be exposed to patient tissue. The segmented electrode also includes an interior surface 586 opposing the stimulation surface 584. The interior surface 566 will be in the interior the lead. One optional feature that aids in anchoring the segmented electrode 552 within the lead is a corrugated, or otherwise rough or non-uniform, texture 588 of the interior surface 586. The non-uniform texture 588 of the interior surface 586 increases the surface area that contacts the material of the lead body that is formed around the segmented electrode 552, as described below, and helps in retaining the segmented electrode within the lead. The corrugation of the texture 588 can have a triangular cross-section, as illustrated in Figure 5, or any other suitable shape including, but not limited, a square, rectangular, trapezoidal, hemispherical, hexagonal, or any other regular or irregular cross-section. Other examples of suitable non-uniform textures include, but are not limited to, a checkerboard arrangement that is similar to corrugation but with intersecting grooves, an arrangement with multiple cleat-like projections or dimples extending from the surface 586, or a surface with a texture formed by knurling, grit blasting, or other methods of roughening of the surface, and the like.

Another optional feature of the segmented electrode 552 is one or more anchoring legs 590. The anchoring legs 590 are arranged so that they project into the interior of the lead and into the material of the lead body that is formed around the segmented electrode. The anchoring legs can have any suitable size or shape and may optionally include one or more holes 592 in the legs. In at least some embodiments, material from the lead body may flow into the holes 592 during the molding process to provide additional anchoring When the segmented electrode 552 includes more than one anchoring leg 590, the anchoring legs may be arranged around the segmented electrode in any suitable arrangement. For example, as illustrated in Figure 5, two anchoring legs 590 may extend from opposing sides towards each other. In other embodiments, the two anchoring legs may extend from only a portion of a particular side of the segmented electrode 552. For example, two anchoring legs may extend from the segmented electrode 552 with one leg extending near one end of a side of the electrode and the other leg extending near the other end of the opposing side of the electrode so that the two legs are diagonally opposed. It will be understood that other arrangements can be used including, for example, arrangements in which legs are directly opposed.

Yet another optional feature of the segmented electrodes 452 is one or more radial channels 494 as illustrated in Figure 4. These radial channels 494 can be on the edges of the segmented electrode 452, as illustrated in Figure 4, or be openings through the body of the segmented electrode These radial channels 494 can facilitate retention of the segmented electrode in the lead body by interacting with the material of the lead body.

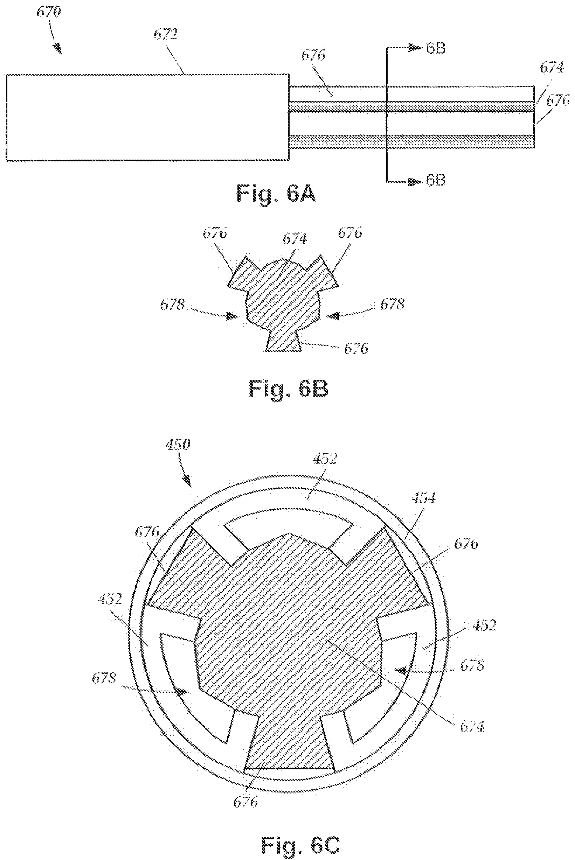

In at least some embodiments, the segmented electrodes 452 can be arranged in the ring 450 using a tool. One embodiment of a suitable tool is the tool 670 illustrated in Figures 6A-6C. The tool 670 includes a handle 672, a central body 674, and projections 676 extending away from the central body 674, as illustrated in Figures 6A and 6B. The regions between the projections 676 form channels 678 that are sized to receive the electrodes 452 so that they can be placed in the ring 454 in the desired arrangement, as illustrated in Figure 6C. In at least some embodiments, the ring 454 can be slid onto the tool. One or more segmented electrodes 452 can then be placed in the channels 678 and slid into the ring 454. The 452 can then be attached to the ring 454 by, for example, welding. The tool 670 can then be rotated and the process repeated for another electrode, and so on.

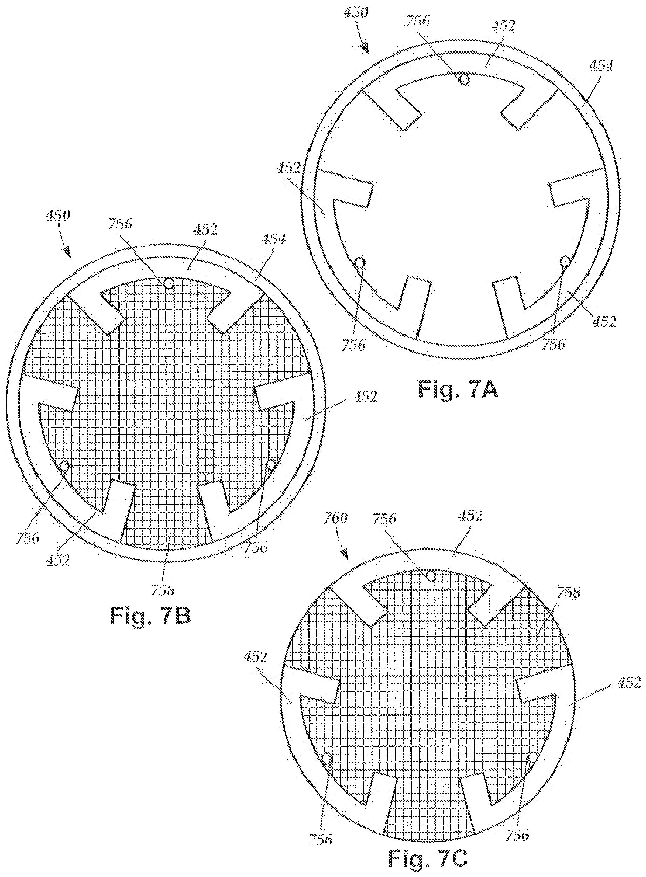

After all of the electrodes 452 are attached to the ring 454, the tool 670 can be removed. Conductor wires 756 can then be coupled to each of the segmented electrodes 452, as illustrated in Figure 7A. The conductor wires can be attached using any suitable technique including, but not limited to, welding, soldering, crimping, staking, or the like.

The lead body 758 can then be formed around the segmented electrodes 452 and conductor wires 756, as illustrated in Figure 7B. The lead body can be formed using, for example, a polymeric material such as polyurethane, silicone, or the like or any combination thereof. It will be understood that there may be more than one ring 454 with segmented electrodes 452 and that the lead body 758 may be simultaneously or sequentially formed around all of these segmented electrodes. For example, in at least some embodiments, one or more rings 454 with segmented electrodes 452 may be placed in a mold in a space-apart arrangement. The material of the lead body 758 can then be molded around all of the segmented electrodes 452 and through each of the rings 454 simultaneously. The material of the lead body 758 may also pass through the holes 592 (see Figure 5), if any, of the segmented electrodes 452 to facilitate retention of the segmented electrodes in contact with the lead body. It will be understood that ring electrodes, such as those illustrated in Figures 3A-3D may also be placed in the mold and the lead body molded through the ring electrodes.

After forming the lead body 758, at least a portion of the ring 454 that connects the segmented electrodes 452 together (and, at least in some embodiments, all, or almost all, of the ring) is removed, as illustrated in Figure 7C. This removal separates the segmented electrodes 452 and also exposes the outer surface of the segmented electrodes so that outer surface can be used for electrical stimulation of adjacent tissue when the lead is implanted. Any suitable process can be used for removing the ring 454, or portions of the ring, including, but not limited to, grinding (such as, centerless grinding), ablation, etching, machining, and the like or any combination thereof. In some embodiments, removal of the ring, or portions of the ring, may also include removal of outer portions of the segmented electrodes 452 or lead body 758 or both.

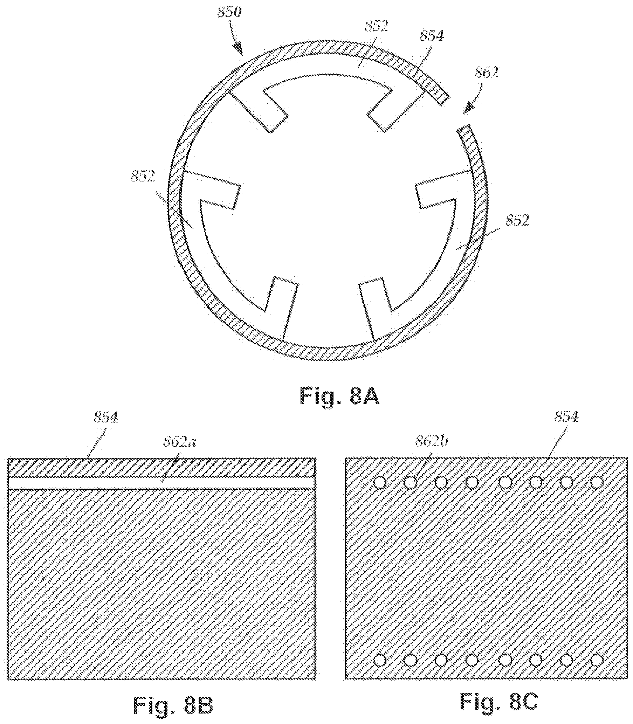

Figures 8A-8C illustrated other embodiments of a pre-electrode 850 with a ring 854 with electrodes 852 attached to the interior of the ring. In these embodiments, the ring 850 has at least one opening 862 through the ring. In Figure 8B, the opening is a slit 862a that can extend the entire axial length of the ring 854 or only a portion of the axial length of the ring. In Figure 8C, the opening is one or more holes 862b formed through the ring 854. The opening 862 (such as slit 862a or hole 862b) may facilitate manufacture as the material of the lead body may extend into the opening as the lead body is formed which may reduce rotational or axial slippage of the ring during subsequent processing (at least until the ring is removed) and, therefore, reduce the possibility of the placement of the segmented electrodes being altered during that processing.

The above specification, examples and data provide a description of the manufacture and use of the invention. Since many embodiments of the invention can be made without departing from the scope of the invention, the invention also resides in the claims heremafter appended.

## Claims

1. A method of making an electrical stimulation lead, the method comprising:
a) attaching a plurality of segmented electrodes to an interior of a ring in a circumferentially spaced-apart arrangement, wherein the ring defines a plurality of holes extending from the interior to an exterior of the ring;
b) attaching a conductor wire to each of the segmented electrodes;
c) coupling the ring with the segmented electrodes to a lead body; and
d) after coupling to the lead body, removing at least those portions of the ring between the segmented electrodes to separate the plurality of segmented electrodes from each other.

2. The method of claim 1, wherein attaching a plurality of segmented electrodes comprises attaching the plurality of segmented electrodes to the interior of the ring in an evenly spaced-apart arrangement.

3. The method of claim 1, further comprising placing the plurality of segmented electrodes into channels in an alignment tool, wherein a portion of the alignment tool containing the channels is disposed within the interior of the ring.

4. The method of claim 3, further comprising removing the alignment tool after the plurality of segmented electrodes are coupled to the interior of the ring.

5. The method of claim 1, wherein attaching a plurality of segmented electrodes comprises welding the plurality of segmented electrodes to the interior of the ring.

6. The method of claim 1, wherein attaching a plurality of segmented electrodes comprises adhesively attaching the plurality of segmented electrodes to the interior of the ring.

7. The method of claim 1, wherein removing at least those portions of the ring between the segmented electrodes comprises grinding the ring to remove the portions of the ring between the segmented electrodes.

8. The method of claim 1, wherein removing at least those portions of the ring comprises removing all of the ring.

9. The method of claim 1, further comprising performing steps a)-d) for at least one additional plurality of segmented electrodes, each plurality of segmented electrodes being attached to a different ring and spaced apart axially from each other along the lead body.

10. A pre-electrode, comprising:
a ring having an interior and defining a plurality of holes extending from the interior to an exterior of the ring; and
a plurality of segmented electrodes attached to the interior of the ring in a circumferentially spaced-apart arrangement.

11. The pre-electrode of claim 10, wherein the plurality of segmented electrodes are attached to the interior of the ring in an evenly spaced-apart arrangement.

12. The pre-electrode of claim 10, wherein the plurality of segmented electrodes are welded to the interior of the ring.

13. The pre-electrode of claim 10, wherein the plurality of segmented electrodes are adhesively attached to the interior of the ring.

14. A method of making the pre-electrode of claim 10, the method comprising:
placing a portion of tool in the ring, the portion of the tool defining a plurality of channels for receiving the segmented electrodes;
individually inserting the plurality of segmented electrodes into the channels of the tool and sliding the segmented electrodes into the ring;
attaching the plurality of segmented electrodes to an interior of the ring in a circumferentially spaced-apart arrangement defined by the tool; and
removing the tool.

15. The method of claim 14, wherein attaching the plurality of segmented electrodes comprises welding or adhesively attaching the plurality of segmented electrodes to the interior of the ring.

## Patentansprüche

1. Verfahren zum Herstellen einer elektrischen Stimulationsleitung, wobei das Verfahren aufweist:
a) Anbringen mehrerer segmentierter Elektroden an einer Innenseite eines Rings in voneinander in Umfangsrichtung beabstandeter Anordnung, wobei der Ring mehrere Löcher definiert, die sich von der Innenseite zu einer Außenseite des Rings erstrecken;
b) Anbringen eines Leitungsdrahts an jeder der segmentierten Elektroden;
c) Ankoppeln des Rings mit den segmentierten Elektroden an einen Leitungskörper; und
d) nach Ankopplung an den Leitungskörper Entfernen von mindestens jenen Teilen des Rings zwischen den segmentierten Elektroden, um die mehreren segmentierten Elektroden voneinander zu trennen.

2. Verfahren nach Anspruch 1, wobei das Anbringen mehrerer segmentierter Elektroden das Anbringen der mehreren segmentierten Elektroden in gleichmäßigem Abstand voneinander an die Innenseite des Rings aufweist.

3. Verfahren nach Anspruch 1, ferner aufweisend das Einsetzen der mehreren segmentierten Elektroden in Kanäle in einem Ausrichtwerkzeug, wobei ein Teil des die Kanäle enthaltenden Ausrichtwerkzeugs im Innenraum des Rings angeordnet ist.

4. Verfahren nach Anspruch 3, das ferner das Entfernen des Ausrichtwerkzeugs aufweist, nachdem die mehreren segmentierten Elektroden an die Innenseite des Rings gekoppelt sind.

5. Verfahren nach Anspruch 1, wobei das Anbringen von mehreren segmentierten Elektroden das Anschweißen der mehreren segmentierten Elektroden an die Innenseite des Rings aufweist.

6. Verfahren nach Anspruch 1, wobei das Anbringen von mehreren segmentierten Elektroden das Ankleben der mehreren segmentierten Elektroden an die Innenseite des Rings aufweist.

7. Verfahren nach Anspruch 1, wobei das Entfernen von mindestens jenen Teilen des Rings zwischen den segmentierten Elektroden das Schleifen des Rings zum Entfernen der Teile des Rings zwischen den segmentierten Elektroden aufweist.

8. Verfahren nach Anspruch 1, wobei das Entfernen von mindestens jenen Teilen des Rings das Entfernen des ganzen Rings aufweist.

9. Verfahren nach Anspruch 1, ferner aufweisend das Ausführen der Schritte a)-d) für mindestens einen zusätzlichen Satz von mehreren Elektroden, wobei jeder Satz von mehreren Elektroden an einem anderen Ring angebracht und in Achsrichtung beabstandet auf dem Leitungskörper angeordnet ist.

10. Vorelektrode, aufweisend:
einen Ring mit einer Innenseite, der mehrere Löcher definiert, die sich von der Innenseite zu einer Außenseite des Rings erstrecken; und
mehrere segmentierte Elektroden, die in einer Umfangsrichtung voneinander beabstandet an der Innenseite des Rings angebracht sind.

11. Vorelektrode nach Anspruch 10, wobei die mehreren segmentierten Elektroden in gleichmäßigem Abstand voneinander an der Innenseite des Rings angebracht sind.

12. Vorelektrode nach Anspruch 10, wobei die mehreren segmentierten Elektroden an die Innenseite des Rings angeschweißt sind.

13. Vorelektrode nach Anspruch 10, wobei die mehreren segmentierten Elektroden an die Innenseite des Rings angeklebt sind.

14. Verfahren zum Herstellen der Vorelektrode nach Anspruch 10, wobei das Verfahren aufweist:
Einsetzen eines Teils des Werkzeugs in den Ring, wobei der Teil des Werkzeugs mehrere Kanäle zum Aufnehmen der segmentierten Elektroden definiert;
einzelnes Einsetzen der mehreren segmentierten Elektroden in die Kanäle des Werkzeugs und Einschieben der segmentierten Elektroden in den Ring;
Anbringen der mehreren segmentierten Elektroden an eine Innenseite des Rings in einer von dem Werkzeug definierten, in Umfangsrichtung voneinander beabstandeten Anordnung; und
Entfernen des Werkzeugs.

15. Verfahren nach Anspruch 14, wobei das Anbringen der mehreren segmentierten Elektroden das Anschweißen oder Ankleben der mehreren segmentierten Elektroden an die Innenseite des Rings aufweist.

## Revendications

1. Procédé de production d'un conducteur de stimulation électrique, le procédé comprenant :
a) la fixation d'une pluralité d'électrodes segmentées à un intérieur d'un anneau dans un agencement séparé suivant la circonférence, où l'anneau définit une pluralité de trous s'étendant depuis l'intérieur jusqu'à un extérieur de l'anneau ;
b) la fixation d'un fil conducteur à chacune des électrodes segmentées ;
c) le couplage de l'anneau avec les électrodes segmentées à un corps de conducteur ; et
d) après le couplage au corps de conducteur, le retrait d'au moins ces parties de l'anneau entre les électrodes segmentées pour séparer la pluralité d'électrodes segmentées les unes des autres.

2. Procédé selon la revendication 1, où la fixation d'une pluralité d'électrodes segmentées comprend la fixation de la pluralité d'électrodes segmentées à l'intérieur de l'anneau dans un agencement séparé uniformément.

3. Procédé selon la revendication 1, comprenant en outre la mise en place de la pluralité d'électrodes segmentées dans des canaux dans un outil d'alignement, où une partie de l'outil d'alignement contenant les canaux est disposée à l'intérieur de l'anneau.

4. Procédé selon la revendication 3, comprenant en outre le retrait de l'outil d'alignement après le couplage de la pluralité d'électrodes segmentées à l'intérieur de l'anneau.

5. Procédé selon la revendication 1, où la fixation d'une pluralité d'électrodes segmentées comprend le soudage de la pluralité d'électrodes segmentées à l'intérieur de l'anneau.

6. Procédé selon la revendication 1, où la fixation d'une pluralité d'électrodes segmentées comprend la fixation de manière adhésive de la pluralité d'électrodes segmentées à l'intérieur de l'anneau.

7. Procédé selon la revendication 1, où le retrait d'au moins ces parties de l'anneau entre les électrodes segmentées comprend le meulage de l'anneau pour retirer les parties de l'anneau entre les électrodes segmentées.

8. Procédé selon la revendication 1, où le retrait d'au moins ces parties de l'anneau comprend le retrait de la totalité de l'anneau.

9. Procédé selon la revendication 1, comprenant en outre l'accomplissement des étapes a)-d) pour au moins une pluralité supplémentaire d'électrodes segmentées, chaque pluralité d'électrodes segmentées étant fixée à un anneau différent et séparée axialement de chaque autre le long du corps de conducteur.

10. Pré-électrode, comprenant :
un anneau ayant un intérieur et définissant une pluralité de trous s'étendant depuis l'intérieur jusqu'à un extérieur de l'anneau ; et
une pluralité d'électrodes segmentées fixées à l'intérieur de l'anneau dans un agencement séparé suivant la circonférence.

11. Pré-électrode selon la revendication 10, où la pluralité d'électrodes segmentées sont fixées à l'intérieur de l'anneau dans un agencement séparé uniformément.

12. Pré-électrode selon la revendication 10, où la pluralité d'électrodes segmentées sont soudées à l'intérieur de l'anneau.

13. Pré-électrode selon la revendication 10, où la pluralité d'électrodes segmentées sont fixées de manière adhésive à l'intérieur de l'anneau.

14. Procédé de production de la pré-électrode selon la revendication 10, le procédé comprenant :
la mise en place d'une partie d'outil dans l'anneau, la partie de l'outil définissant une pluralité de canaux pour recevoir les électrodes segmentées ;
l'insertion individuellement de la pluralité d'électrodes segmentées dans les canaux de l'outil et le glissement des électrodes segmentées dans l'anneau ;
la fixation de la pluralité d'électrodes segmentées à un intérieur de l'anneau dans un agencement séparé suivant la circonférence défini par l'outil ; et
le retrait de l'outil.

15. Procédé selon la revendication 14, où la fixation de la pluralité d'électrodes segmentées comprend le soudage ou la fixation de manière adhésive de la pluralité d'électrodes segmentées à l'intérieur de l'anneau.
